# EUROPEAN PATENT SPECIFICATION

(11) **EP 1 410 815 B1**
(45) Date of publication and mention of the grant of the patent: **26.12.2012**
(21) Application number: 03028317.0
(22) Date of filing: 19.04.1996
(51) Int. Cl.: A61M 5/142, A61M 31/00, A61M 5/145

(54) **Self purging angiographic injector**
Selbstreinigender Injektor für die Angiographie
Injecteur autopurgeur pour angiographie

(30) Priority: 20.04.1995 US 425577; 20.04.1995 US 426149
(43) Date of publication of application: 21.04.2004
(62) Divisional of application: 96911818.1
(73) Proprietor: ACIST Medical Systems, Inc., Eden Prairie, MN 55344 (US)
(72) Inventor: Wilson, Robert F., Shoreview, Minnesota 55126 (US); Liu, Jiyan, Maplewood, Minnesota 55109 (US)
(74) Representative: Eisenführ, Speiser & Partner

(56) References cited:
- EP-A- 0 110 276
- WO-A-96/32975
- US-A- 3 559 644
- US-A- 3 957 052
- US-A- 5 095 880

## Description

### BACKGROUND OF THE INVENTION

This invention relates to angiography and more specifically, the injector used to inject a medical fluid such as radiographic contrast material into living organisms.

One of the major systems in the human body is the circulatory system. The major components of the circulatory system are the heart, blood vessels, and the blood, all of which are vital to the transportation of materials between the external environment and the different cells and tissues of the human body.

The blood vessels are the network of passageways through which the blood travels in the human body. Specifically, arteries carry the oxygenated blood away from the left ventricle of the heart. These arteries are aligned in progressively decreasing diameter and pressure capability from the aorta, which carries the blood immediately out of the heart to other major arteries, to smaller arteries, to arterioles, and finally to tiny capillaries, which feed the cells and tissues of the human body. Similarly, veins carry the oxygen depleted blood back to the right atrium of the heart using a progressively increasing diameter network of venules and veins.

If the heart chambers, valves, arteries, veins or other capillaries connected thereto are either abnormal (such as from a birth defect), restricted (such as from atherosclerotic plaque buildup), or deteriorating (such as from aneurism formation), then a physician may need to examine the heart and connected network of vessels. The physician may also need to correct any problems encountered during the examination with a catheter or similar medical instrument.

Angiography is a procedure used in the detection and treatment of abnormalities or restrictions in blood vessels. During angiography, a radiographic image of a vascular structure is obtained by injecting radiographic contrast material through a catheter into a vein or artery. The vascular structures fluidly connected with the vein or artery in which the injection occurred are filled with contrast material. X-rays are passed through the region of the body in which the contrast material was injected. The X-rays are absorbed by the contrast material, causing a radiographic outline or image of the blood vessel containing the contrast material. The x-ray images of the blood vessels filled with contrast material are usually recorded onto film or videotape and are displayed on a fluoroscope monitor.

Angiography gives the doctor an image of the vascular structures in question. This image may be used solely for diagnostic purposes, or the image may be used during a procedure such as angioplasty where a balloon is inserted into the vascular system and inflated to open a stenosis caused by atherosclerotic plaque buildup.

Currently, during angiography, after a physician places a catheter into a vein or artery (by direct insertion into the vessel or through a skin puncture site), the angiographic catheter is connected to either a manual or an automatic contrast injection mechanism.

A simple manual contrast injection mechanism typically has a syringe and a catheter connection. The syringe includes a chamber with a plunger therein. Radiographic contrast material is suctioned into the chamber. Any air is removed by actuating the plunger while the catheter connection is facing upward so that any air, which floats on the radiographic contrast material, is ejected from the chamber into the air. The catheter connection is then attached to a catheter that is positioned in a vein or artery in the patient.

The plunger is manually actuated to eject the radiographic contrast material from the chamber, through the catheter, and into a vein or artery. The user of the manual contrast injection mechanism may adjust the rate and volume of injection by altering the manual actuation force applied to the plunger.

Often, more than one type of fluid injection is desired, such as a saline flush followed by the radiographic contrast material. One of the most common manual injection mechanisms used today includes a valve mechanism which controls which of the fluids will flow into the valving mechanism and out to the catheter within the patient. The valve mechanism contains a plurality of manual valves that the user operates manually to open and close that particular fluid channel. When the user suctions or injects contrast fluid into the chamber, the fluid is pulled from the valve mechanism via the open valves. By changing the valve positions, another fluid may be injected.

These manual injection mechanisms are typically hand actuated. This allows user control over the quantity and pressure of the injection. However, all of the manual systems are only capable of injecting the radiographic contrast material at maximum pressure that can be applied by the human hand (i.e., 150 p.s.i & i.e. 10,5 bar). Also, the quantity of radiographic contrast material is typically limited to a maximum of about 12cc. Finally, there are no safety limits on these manual contrast injection mechanisms which act to restrict or stop injections that are outside of reasonable parameters (such as rate or pressure) and no active sensors to detect air bubbles or other hazards.

Currently used motorized injection devices consist of a syringe connected to a linear actuator. The linear actuator is connected to a motor, which is controlled electronically. The operator enters into the electronic control a fixed volume of contrast material to be injected at a fixed rate of injection. The fixed rate of injection consists of a specified initial rate of flow increase and a final rate of injection until the entire volume of contrast material is injected. There is no interactive control between the operator and machine, except to start or stop the injection. Any change in flow rate must occur by stopping the machine and resetting the parameters.

The lack of ability to vary the rate of injection during the injection results in suboptimal quality of angiographic studies. This is because the optimal flow rate of injections varies considerably between patients. In the cardiovascular system, the rate and volume of contrast injection is dependent on the size of and blood flow rate within the chamber or blood vessel being injected. In many or most cases, these parameters are not known precisely. Moreover, the optimal rate of injection can change rapidly, as the patient's condition changes in response to drugs, illness, or normal physiology. Consequently, the initial injection of contrast material may be insufficient in flow rate to outline the structure on x-ray imaging, necessitating another injection. Conversely, an excessive flow rate might injure the chamber or blood vessel being injected, cause the catheter to be displaced (from the jet of contrast material exiting the catheter tip), or lead to toxic effects from contrast overdose (such as abnormal heart rhythm).

At present, the operator can choose between two systems for injecting contrast material: a manual injection system which allows for a variable, operator interactive flow rate of limited flow rate and a preprogrammed motorized system without operator interactive feedback (other than the operator can start/stop the procedure).

### SUMMARY OF THE INVENTION

The present invention is an apparatus for delivering medical fluids such as angiographic radiographic contrast material to a patient. In one preferred embodiment, the apparatus is a dual port syringe which preferably features a self purging operation to eliminate air. The apparatus of the present invention includes an inlet port for connection to a fluid reservoir, a syringe and a valve connected in a flow path between the supply port and the syringe. During a fill operation, the medical fluid is drawn from the fluid reservoir through the first valve and into the syringe by rearward movement of the syringe plunger or piston. During a purge operation, the piston is moved forward to expel air through the first valve to the inlet port. The first valve permits air flow through the flow path from the syringe to the inlet port, but blocks flow of medical fluid back to the inlet port.

The apparatus also include, and preferred embodiments, a second valve which is connected between the syringe and the patient. The second valve is in a closed state during the fill and air purge operation. When an injection is to be performed, the second valve opens to permit flow of the medical fluid under pressure from the syringe to the patient. At the same time, the first valve prevents flow of a medical fluid from the syringe to the inlet port. The preferred dual port syringe includes a syringe body, a piston which is reciprocally movable in the syringe body, and upper and lower ports. The upper port is connected to a fluid reservoir so that medical fluid is drawn from the fluid reservoir through the upper port into the syringe body when the piston moves in a rearward direction. The lower port is connected to a device, such as a catheter, through which the medical fluid is delivered under pressure to the patient. When the piston moves in a forward direction, medical fluid is delivered under pressure out of the syringe body through the lower port.

### BRIEF DESCRIPTION OF THE DRAWINGS

Fig. 1 is a perspective view illustrating a preferred embodiment of the angiographic injector system of the present invention.
Figs. 2A-2G are diagrams illustrating operations of the system of Fig. 1.
Fig. 3 is an electrical block diagram of the control system of the injector system of Fig. 1.
Fig. 4 illustrates front panel controls and displays of a preferred embodiment of the injector system of the present invention.
Figs. 5A and 5B are side and partial top perspective views of the remote control of the system of Fig. 1.

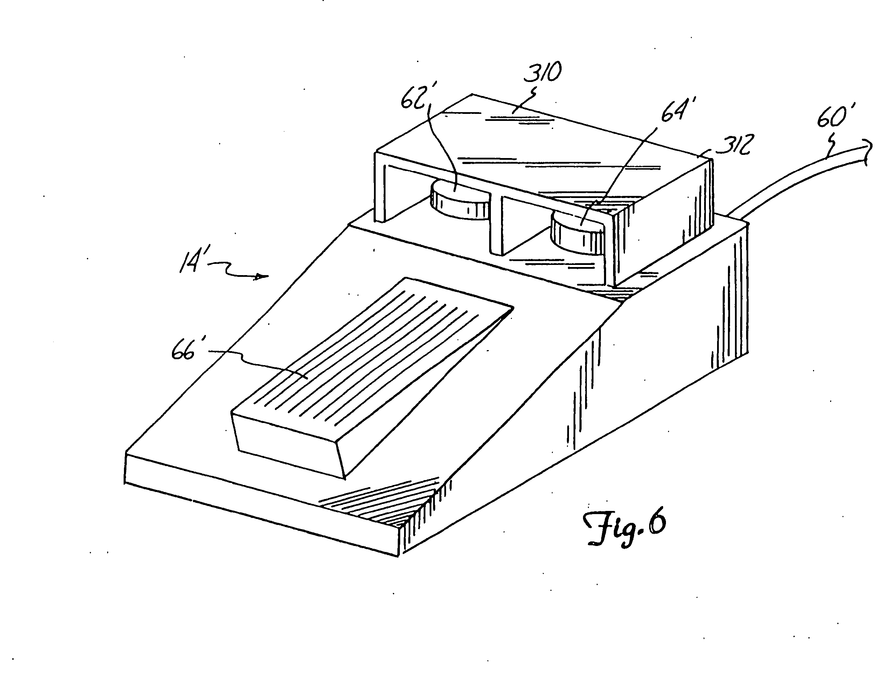

Fig. 6 is a perspective view of a foot operated remote control.
Figs. 7A-7D illustrate the operation of the inlet check valve and manifold during contrast fill, air purge, and patient inject operations.
Figs. 8A-8C illustrate operation of the inlet check valve in greater detail.
Fig. 9 shows a conventional syringe body adapted for dual port operation.

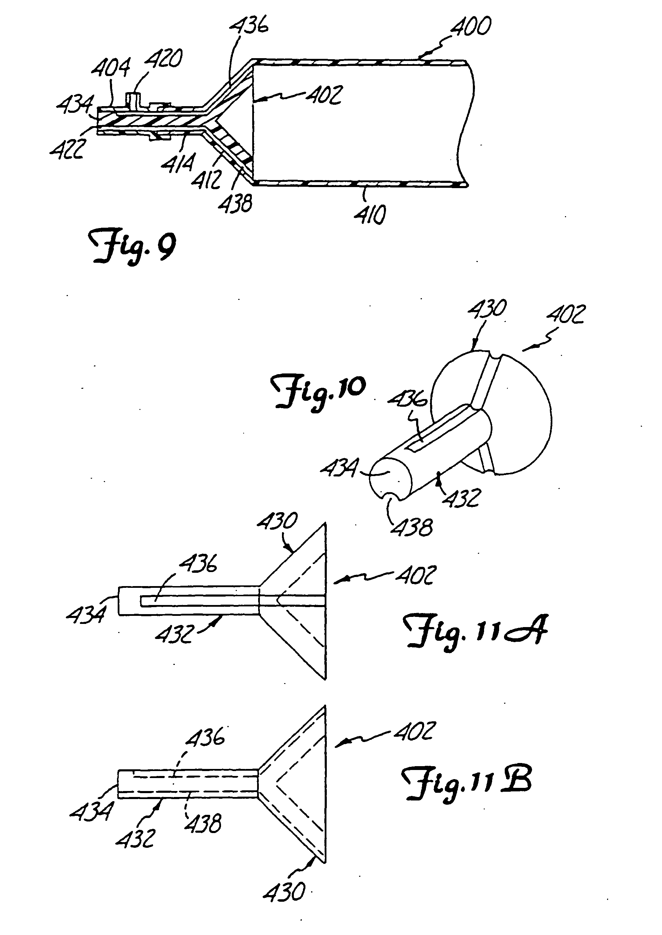

Fig. 9 is a perspective view of an adaptor insert used in the dual port syringe of Fig. 9.
Figs. 11A - 11B are top and side views of the adaptor insert of Fig.
10.

### DETAILED DESCRIPTION OF THE PREFERRED EMBODIMENTS

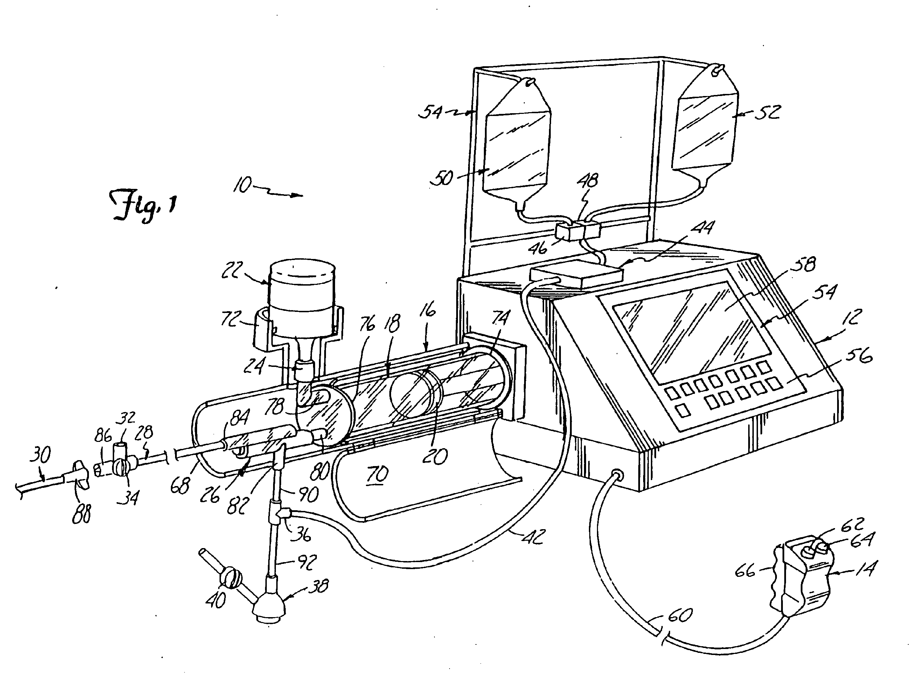

Figure 1 shows angiographic injector system 10 for injecting radiographic contrast material into a blood vessel under interactive physician control. System 10 includes main console 12, hand held remote control 14, syringe holder 16, syringe body 18, syringe plunger 20, radiographic material reservoir (bottle) 22, one-way valve 24, manifold 26, high pressure tube 28, catheter 30, patient medication port 32, three-way stop-cock 34, T-connector 36, pressure transducer 38, stop-cock 40. tubing 42, peristaltic pump 44, saline check valve 46, waste check valve 48. saline bag 50, waste bag 52, and bag support rack 54.

Console 12 houses the electrical controls for system 10, together with the motors which drive piston 20 and peristaltic pump 44. On the front surface of console 12, user interface 54 provides control switches 56 and display 58 through which the user may enter control settings and monitor the operational state of system 10.

Remote control 14 is connected to console 12 by cable 60 (although in other embodiments remote control 14 may be connected by a wireless connection such as an RF, infrared, optic, or ultrasonic link). Remote control 14 is, in the embodiment shown in Fig. 1, a hand-held control which includes reset and saline push button switches 62 and 64, respectively, and flow rate control lever or trigger 66. By squeezing trigger 66, the user can provide a command signal to console 12 to provide a continuously variable injection rate.

Syringe holder 16 projects from the left hand side of console 12. Syringe holder 16 is preferably a clear material, and includes a half cylindrical back shell 68, a half cylindrical front door 70 (which is shown in open position in Fig. 1), and reservoir holder 72.

Syringe 18 is a transparent or translucent plastic cylinder having its open end 74 connected to console 12. Closed end 76 of syringe 18 contains two ports: upper port 78 and lower port 80.

Plunger 20 is movable within syringe body 18. Plunger 20 is connected to, and driven by a motor located within console 12.

Radiographic contrast material reservoir 22 is connected through one-way check valve 24 to upper port 78. Radiographic contrast material is drawn from reservoir 22 through check valve 24 and upper port 78 into the pumping chamber defined by syringe body 18 and plunger 20. Check valve 24 is preferably a weighted one-way valve which permits air to flow from syringe body 18 back into reservoir 22, but will not permit radiographic contrast material to flow from syringe body 18 to reservoir 22. This permits automatic purging of air from the system, as will be described in more detail later.

Lower port 80 of syringe body 18 is connected to manifold 26. Manifold 26 includes a spring biased spool valve which normally connects transducer/saline port 82 and patient port 84. When radiographic contrast material is to be injected, the pressure of the radiographic material causes the spool valve to change states so that lower port 80 is connected to patient port 84.

High pressure tube 28 is a flexible tube which connects patient port 84 to catheter 30. Three-way stop-cock 34 is located at the distal end of tube 28. Rotatable luer lock connector 86 is connected to stop-cock 34 and mates with luer connector 88 at the proximal end of catheter 30. Stop-cock 34 either blocks flow between tube 28 and catheter 30, permits flow, or connects medication port 32 to catheter 30.

In addition to injecting radiographic material into a patient through catheter 30, system 10 also permits other related functions to be performed. A device for delivering the patient medication (not shown in Fig. 1) may be connected to medication port 32 when medication is to be delivered through catheter 30 to the patient.

When catheter 30 is in place in the patient, and an injection of radiographic contrast material is not taking place, pressure transducer 38 monitors the blood pressure through the column of fluid which extends from catheter 30, tube 28, patient port 84, manifold 26, transducer/saline port 82, tubing 90, T-connector 36, and tubing 92. Transducer 38 has an associated stop-cock 40 which allows transducer 38 to be exposed to atmospheric pressure during calibration and also allows for removal/expulsion of trapped air so the dome chamber of transducer 38 can be flushed with saline.

Peristaltic pump 44 supplies saline solution from bag 50 through saline check valve 46, tubing 42, T-connector 36 and tubing 90 to saline port 82. When peristaltic pump 44 is operating to supply saline solution, the saline solution is supplied through manifold 26 to patient port 84 and then through tube 28 to catheter 30.

Peristaltic pump 44 also operates in an opposite direction to draw fluid from catheter 30 and through tube 28, manifold 26, tubing 90, T-connector 36 and tubing 42 to waste check valve 48 and then into waste collection bag 52.

In a preferred embodiment of the present invention, syringe body 18, manifold 26, tube 28, catheter 30, T-connector 36, tubing 42, check valves 46 and 48, bags 50 and 52, and tubing 90 and 92 are all disposable items. They must be installed in system 10 each time an angiography procedure is to be performed with a new patient. Once system 10 is set up with all the disposable items installed, door 70 is closed, and syringe body 18 filled with contrast material and purged of air, the user (typically a physician) enters into system 10 the safety parameters that will apply to the injection of radiographic contrast material. These safety parameters typically include the maximum amount of radiographic contrast material to be injected during any one injection, the maximum flow rate of the injection, the maximum pressure developed within syringe body 18, and the maximum rise time or acceleration of the injection. To actuate an injection of contrast material, the user operates remote control 14 by squeezing trigger 66. Within the preset safety parameters, system 10 causes the flow rate of the injection to increase as the force or distance of travel of trigger 66 is increased.

Typically, the user will meter the amount and rate of contrast material injected based upon continuous observation of the contrast outflow into the structure being injected using fluoroscopy or other imaging methods. System 10 allows the user to tailor the contrast injections to the needs of the patient, thereby maximizing the quality of the procedure, increasing the safety, and reducing the amount of contrast material required to perform the fluoroscopic examination.

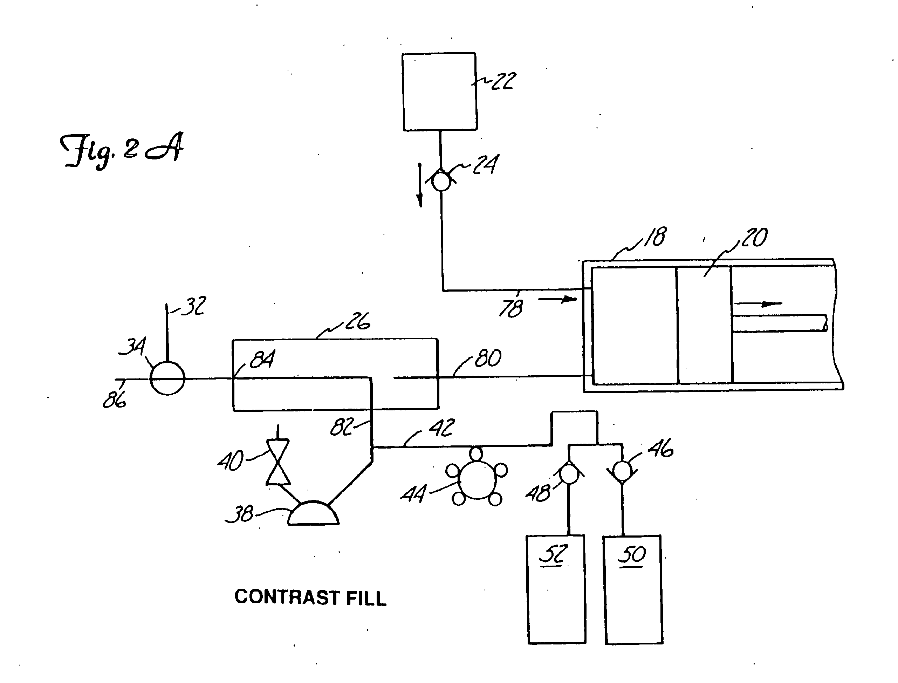

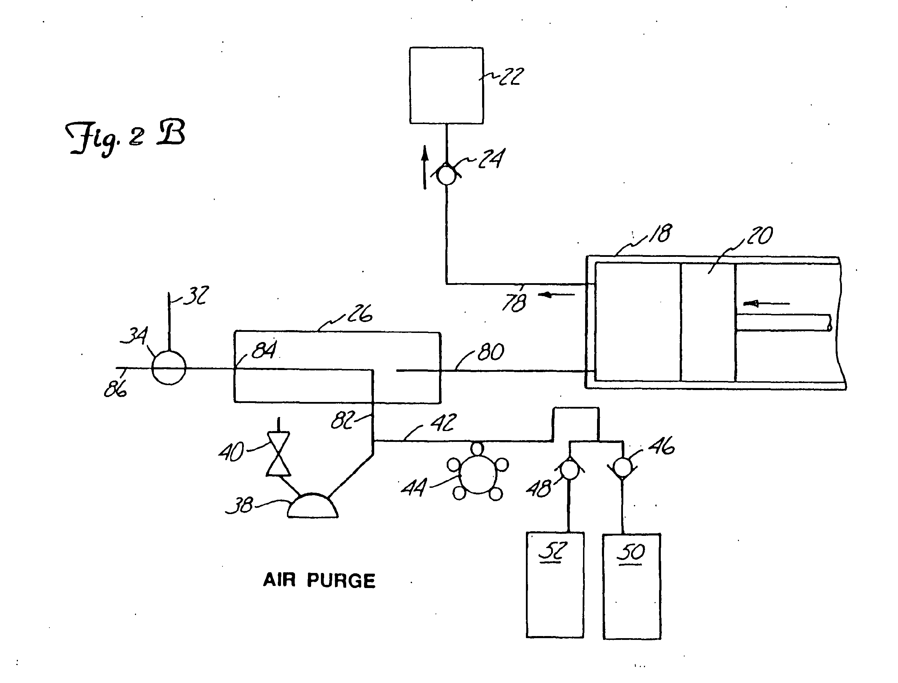

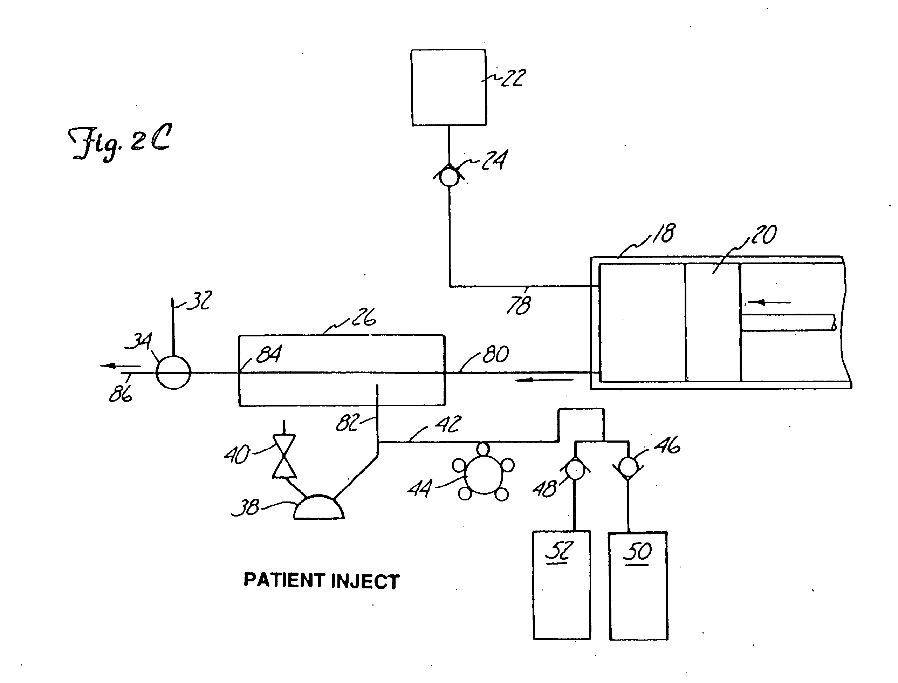

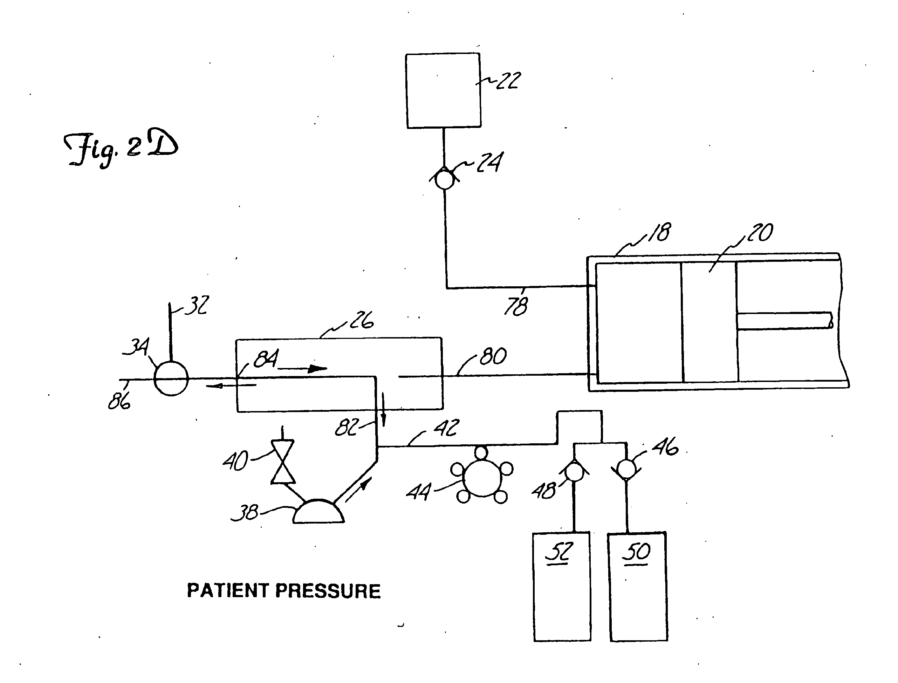

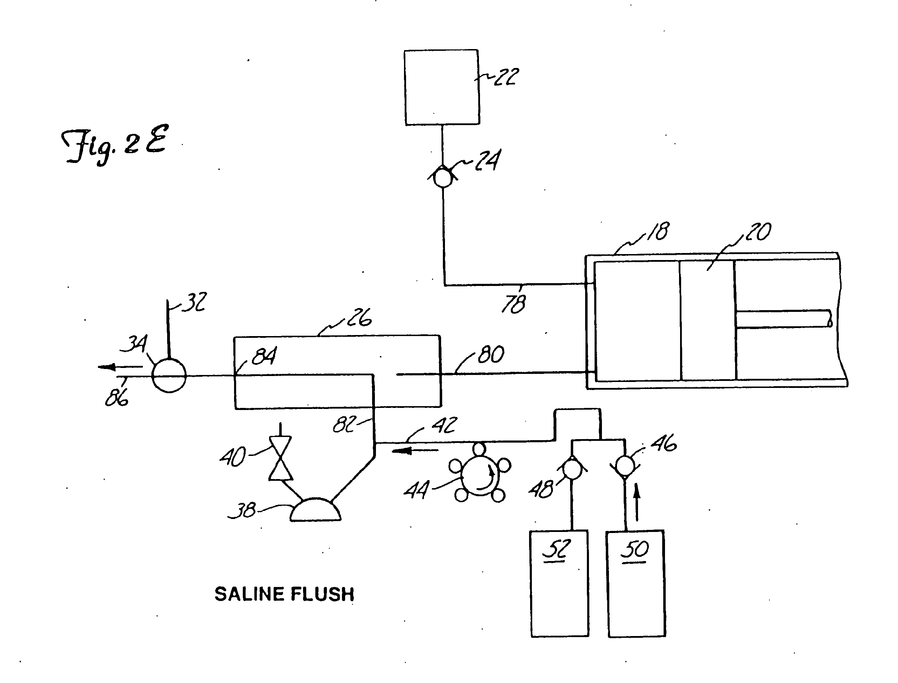

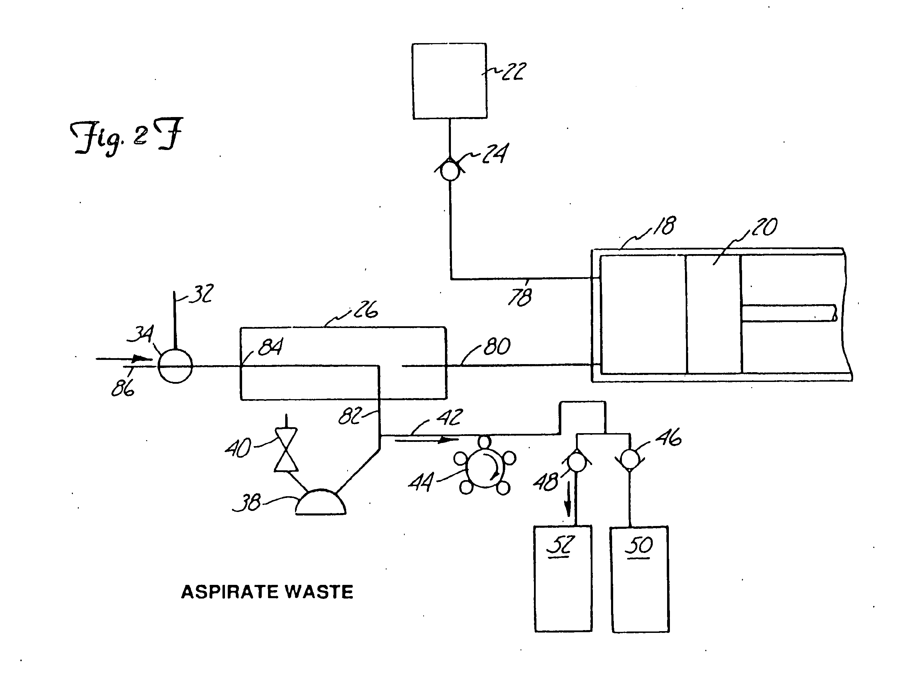

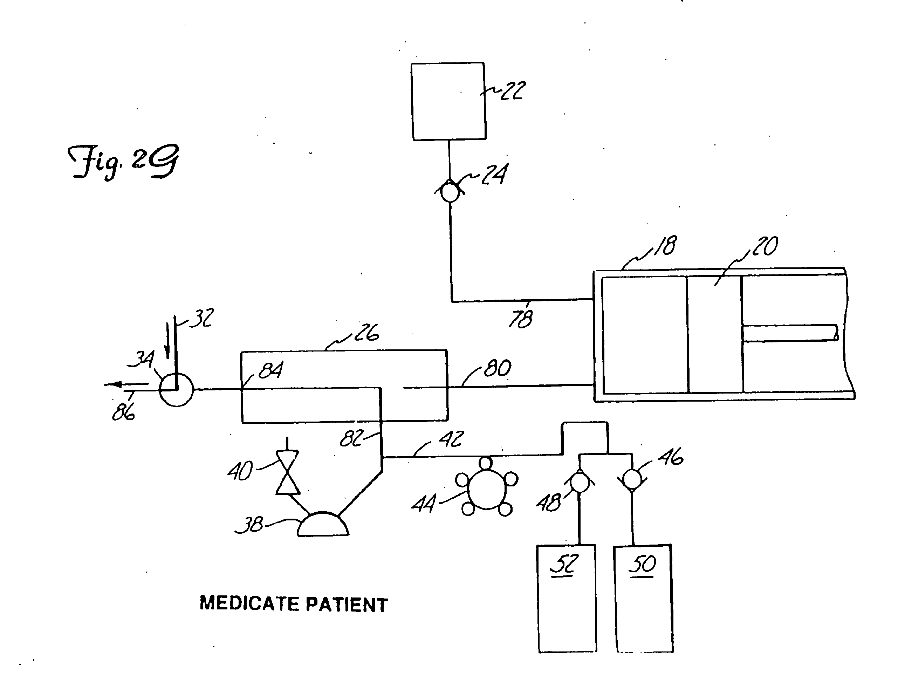

Figs. 2A-2G are diagrams illustrating fluid flow paths during seven different operations of system 10. Those operations are contrast fill (Fig. 2A), air purge (Fig. 2B), patient inject (Fig. 2C), patient pressure (Fig. 2D). saline flush (Fig. 2E), aspirate waste (Fig. 2F), and medicate patient (Fig. 2G).

The contrast fill operation illustrated in Fig. 2A involves the filling of syringe body 18 with radiographic contrast material from reservoir (contrast media supply) 22. The contrast fill operation is performed during initial set up of system 10, and may be repeated during operation of system 10 whenever syringe body 18 is running low on radiographic contrast material.

During initial set up of system 10, plunger 20 is initially driven to its furthest forward position adjacent closed end 76 of syringe body 18. This will expel to the atmosphere the majority of the air which is located within syringe body 18.

Plunger 20 is then retracted, which creates a vacuum within syringe body 18 which draws contrast material from reservoir 22 through check valve 24 into syringe body 18 through upper port 78.

The Contrast Fill operation typically will result in some air being drawn into or remaining within syringe body 18. It is important, of course, to prevent air from being injected into the patient through catheter 30. That is the purpose of the Air Purge operation shown in Fig. 2B. Also, the location of two ports at different elevations allows for a greater amount of safety in preventing air bubbles in the injection.

During the Air Purge operation, plunger 20 travels forward to expel trapped air within syringe body 18. The air, being lighter than the contrast material, gathers near the top of syringe body 18. As plunger 20 moves forward, the air is expelled from syringe body 18 through upper port 78 and one-way valve 24. In the embodiment illustrated in Fig. 2B, one-way valve 24 is a weighted one-way valve which allows flow of radiographic contrast material from reservoir 22 to upper port 78, but will not allow radiographic contrast material to flow in the opposite direction from upper port 78 to reservoir 22. Valve 24 will, however, allow air to flow from port 78 to reservoir 22. As soon as radiographic contrast material begins flowing out of syringe body 18 through upper port 78 to valve 24, valve 24 closes to prevent any further flow toward reservoir 22.

Valve 24 can also, in alternative embodiments, can be a solenoid actuated or motor driven valve operated under control of the electric circuitry within console 12. In either case, valve 24 is capable to withstanding the relatively high pressures to which it will be subjected during the inject operation. Preferably, valve 24 is capable of withstanding static fluid pressures up to about 1200 p.s.i.

Fig. 2C illustrates the Patient Inject operation. Plunger 20 travels forward under the interactive control of the user, who is controlling trigger 66 of remote control 14. The movement of plunger 20 creates hydraulic pressure to force contrast material out of syringe body 18 through lower port 80 and through manifold 26 and high pressure tube 28 into catheter 30. As shown in Fig. 2C, syringe lower port 80 and patient port 84 are connected for fluid flow during the patient inject operation.

Manifold 26 contains a valve which controls the routing of fluid connections between patient port 84 and either syringe bottom port 80 or transducer/saline port 82. In one embodiment of the present invention, manifold 26 includes a spool valve which is spring biased so that patient port 84 is normally connected to transducer/saline port 82 (as illustrated in Figs. 2A and 2B). When the pressure at syringe bottom port 80 builds with the movement of plunger 20 forward, the bias force against the spool valve is overcome so that syringe bottom port 80 is connected to patient port 84, and transducer/saline port 82 is disconnected the valve within manifold 26 protects pressure transducer 38 from being exposed to the high pressure generated by the patient inject operation.

The spool valve opens automatically during the patient inject operation in response to increase pressure exerted on it from the syringe lower port 80. The spool valve closes and returns to its original position allowing for connection of patient port 84 to transducer 38 when a slight vacuum is applied by retraction of plunger 20 at the end of each Patient Inject operation.

In an alternative embodiment, the valve within manifold 26 is an electromechanical or motor driven valve which is actuated at appropriate times to connect either syringe lower port 80 or transducer/saline port 82 to patient port 84. The actuator mechanism is controlled by console 12. Once again in this alternative embodiment, the valve protects pressure transducer 38 from being exposed to high pressure.

Fig. 2D illustrates the Patient Pressure operation. System 10 allows for reading of the patient's blood pressure, which is monitored through catheter 30. Patient blood pressure can be monitored through the use of pressure transducer 38 at any time except during the patient inject, saline flush, and waste aspirate operations. The pressure reading being produced by pressure transducer 38 may be normalized by manually opening stop-cock 40 and closing stop-cock 34 to expose pressure transducer 38 to atmospheric pressure.

During the Saline Flush operation illustrated in Fig. 2E, saline solution is used to flush all of the internal lines, pressure transducer chamber 38, tube 28, and catheter 30. As shown in Fig. 2E, peristaltic pump 44 is operating in a direction which causes saline solution to be drawn from bag 50 through check valve 46 and through tubing 42 to saline port 82. Manifold 26 connects saline port 82 to patient port 84 so that saline solution is pumped out of patient port 84 and through tube 28 and catheter 30.

During the Aspirate Waste operation, patient port 84 is again connected to saline port 82. During this operation, peristaltic pump 44 is operating in the opposite direction from its rotation during the saline flush operation. As a result, patient fluids are aspirated from patient port 84 to saline port 82 and then through tubing 42 and check valve 48 into waste collection bag 52. Peristaltic pump 44 acts as a valve pinching/occluding tubing 42 and preventing back flow to/from saline and waste containers 50 and 52 in conjunction with check valves 46 and 48.

With catheter 30 in place within the patient, it may be desirable to supply patient medication. System 10 allows for that option by providing patient medication port 32. As shown in Fig. 2G, when stop-cock 34 is open, a medication source connected to port 32 will be connected to patient port 84, and thereby to catheter 30. During the medicate patient operation, peristaltic pump 44 and plunger 20 are not moving.

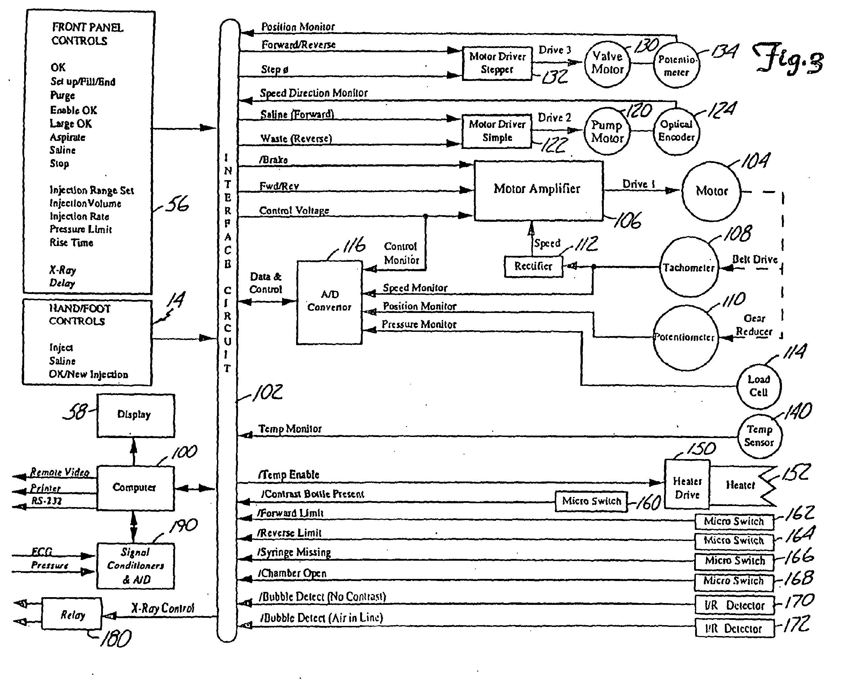

Fig. 3 is an electrical block diagram of the control system which controls the operation of angiographic injector system 10. The electrical control system includes digital computer 100, which receives input signals from remote control 14 and front panel controls 56 through interface 102, and provides signals to display 58 to display operation data, alerts, status information and operator prompts.

Computer 100 controls the motion of plunger 20 through a motor drive circuit which includes motor 104, motor amplifier 106, tachometer 108, potentiometer 110, a rectifier 112, pressure sensing load cell 114, and A/D converter 160.

Motor amplifier 106 provides a Drive 1 signal to motor 104 in response to Control Voltage, Fwd/Rev,and/Brake signals from computer 100 and a speed feedback signal from tachometer 108 through rectifier 112. The outputs of tachometer 108 and potentiometer 110 are supplied to computer 100 through A/D converter 116 as Speed Monitor and Position Monitor signals. These allow computer 100 to check motor speed, motor direction, and position (volume is a calculated value).

Pressure sensor 114 senses motor current or plunger force in order to measure the pressure being applied to the radiographic contrast material within syringe body 18. This Pressure Monitor Signal is supplied through A/D converter 116 and interface 102 to computer 100.

Peristaltic pump 44 is driven under the control of computer 100 through pump motor 120, motor driver 122 and optical encoder 124. Computer 100 provides Saline (Forward) and Waste (Reverse) drive signals to motor driver 122 to operate pump motor 120 in a forward direction for saline flush and a reverse direction for waste aspiration. Optical encoder 124 provides the Speed Direction Monitor signal to interface 102 which indicates both the speed and the direction of rotation of pump motor 120.

Fig. 3 illustrates an embodiment of the control system in which valve motor 130 is used to actuate valves such as one-way valve 24 and the valve within manifold 26. In this embodiment, computer 100 controls valve motor 130 through motor driver 132, and monitors position through a Position Monitor feedback signal from potentiometer 134. In this particular embodiment, valve motor 130 is a stepper motor.

Computer 100 monitors temperature of the contrast material based upon a Temp Monitor signal from temperature sensor 140. Temperature sensor 140 is preferably positioned near syringe body 18. If the temperature being sensed by temperature sensor 140 is too high, computer 100 will disable operation motor 104 to discontinue patient injection. If the temperature is to low, computer 100 provides a /Temp Enable drive signal to heater drive 150, which energizes heater 152. In one preferred embodiment, heater 152 is a resistive film heater which is positioned within syringe holder 116 adjacent to syringe body 18.

Computer 100 also receives feedback signals from contrast bottle sensor 160, forward limit sensor 162, reverse limit sensor 164, syringe missing sensor 166, chamber open sensor 168, no contrast bubble detector 170, and air in line bubble detector 172.

Contrast bottle sensor 160 is a miniature switch located within reservoir holder 72. The state of the Contrast Bottle Present signal from sensor 160 indicates whether a reservoir 22 is in position within holder 72. If reservoir 22 is not present, computer 100 will disable the fill operation.

Forward limit and reverse limit sensors 162 sense the end limit positions of plunger 20. When plunger 20 reaches its forward limit position, no further forward movement of plunger 20 is permitted. Similarly, when reverse limit sensor 164 indicates that plunger 20 has reached its reverse limit position, no further reverse movements are permitted.

Syringe missing sensor 166 is a miniature switch or infrared emitter/detector which indicates when syringe body 18 is not in position within syringe holder 16. If syringe body 18 is not in position, all movement functions are disabled except that plunger 20 can move to its reverse limit position (i.e., return to zero).

Chamber open sensor 168 is a miniature switch or infrared emitter/detector which senses when door 70 of syringe holder 16 is open. When the signal from sensor 168 indicates that door 70 is open, all movement functions are disabled. Only when door 70 is closed and locked may any movement be allowed. When door 70 is indicated as closed and sensor 166 indicates the syringe body 18 is in position, other normal functions of the system 10 can proceed.

Bubble detector 170 is positioned between reservoir 22 and top port 78, and is preferably an infrared emitter/detector which senses air bubbles. If an air bubble is sensed in the flow path between reservoir 22 and top port 78 during a fill operation, the fill operation is disabled until a new reservoir is connected.

Bubble detector 172 is positioned to sense air bubbles in high pressure line 28. It is preferably an infrared emitter/detector type of bubble detector. Any air bubble which is sensed in high pressure line 28 results in the disabling of all fluid push out functions, whether the fluid is saline solution from peristaltic pump 44 or contrast material from syringe body 18.

The control system of Fig. 3 also includes the capability to provide a control signal to x-ray equipment through relay 180 which is controlled by computer 100. In addition, computer 100 receives data from blood pressure transducer 38 and from an electrocardiograph (ECG) system, which is separate from injector system 10. The Pressure and ECG signals are received through signal conditioners and A/D converter 190, and are transferred to computer 100. The ECG signal is used by computer 100 in one preferred embodiment, to synchronize operation of motor 104 (and thus the Patient Inject operation) with heart beats.

Blood flow to the heart occurs predominantly in diastole (when the heart is between contractions). Continuous injection of contrast material results in spillage of the contrast material into the aorta during systole (during contraction). By injecting primarily during diastole, contrast dosage can be reduced without impairing the completeness of the contrast injection into the coronary artery.

In a preferred embodiment, the injection of radiographic contrast material is synchronized to the coronary artery blood flow. The time periods of systole and diastole are determined using an electrocardiographic (ECG) electrical signal, arterial blood pressure waveform analysis, or other timing based on the heart rate. By controlling speed of motor 104, speed and therefore movement of plunger 20, the injection of contrast material is interrupted during the period of systole, which reduces or stops contrast injection during this time. In combination with remote control 14, the operator can vary the rate of contrast injection into the coronary artery while computer 100 automatically pulses the contrast injection to the cardiac cycle

The inertial forces of the moving contrast material and expansion of the containers and tubing holding the contrast material and transmitting it to the patient can cause a phase lag between movement of plunger 20 within syringe body 18 and movement of contrast material out of catheter 30 into the patient. To adjust to the phase lag between the plunger 20 movement and contrast expulsion into the patient, a variable time offset can be entered through control panel 54 such that the timing of the cardiac cycle can be offset by a selected time. Since the magnitude of the phase lag may be dependent on the frequency of the heart rate, an algorithm within computer 100 continuously and automatically adjusts the magnitude of the time offset, based on the instantaneous heart rate during the injection of contrast material.

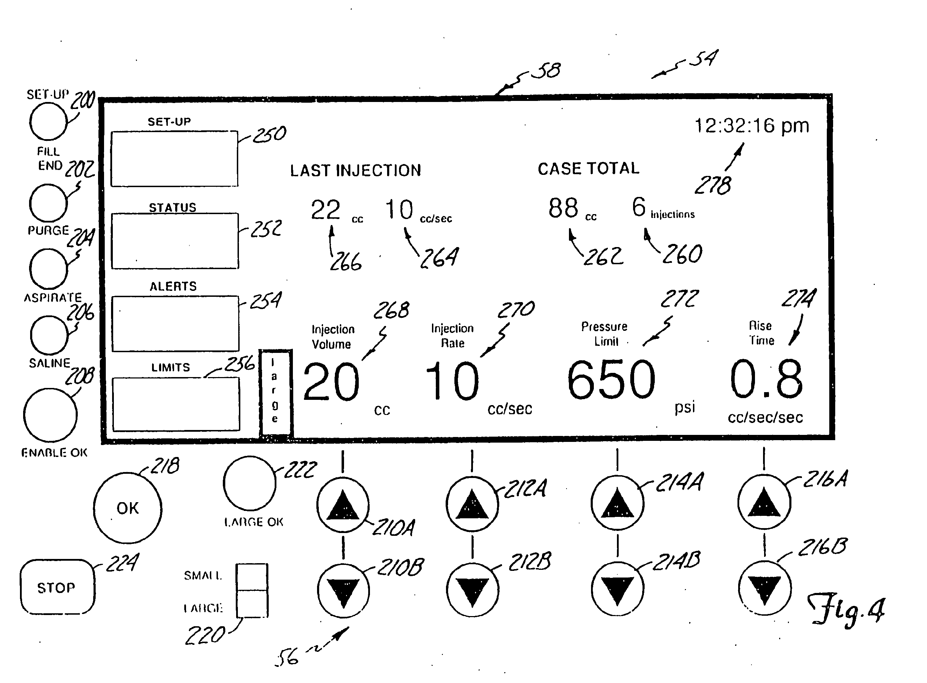

Fig. 4 shows one embodiment of control panel 54 which illustrates the front panel control switches 56 and display 58 of one embodiment of the present invention. Front panel control switches 56 include Set Up/Fill/End switch 200, Purge switch 202, Aspirate switch 204, Saline switch 206, Enable OK switch 208, Injection Volume Limit switches 210a and 210b, Injection Flow Rate Limit switches 212a and 212b, Injection Pressure Limit switches 214a and 214b, Rise Time switches 216a and 216b, OK switch 218, Injection Range Toggle switch 220, Large Injection OK switch 222, and Stop switch 224.

Set Up/Fill/End switch 200 is a momentary push button switch. When it is first activated, the user will be notified to place syringe 18 in syringe holder 16. When syringe 18 has been placed in syringe holder 16 (which is indicated to computer 100 by sensor 166), the user will be instructed to close and lock the chamber (i.e., to close door 70). Plunger 20 is moved to its full forward position expelling all air within the syringe. Display 58 then indicates to the operator that contrast reservoir 22 should be connected. Once contrast reservoir 22 has been put in place, the operator is requested to depress OK switch 218, at which time plunger 20 will retract at a set rate (preferably corresponding to a flow rate of 10 ml per second) to the maximum syringe volume. If the real speed (as indicated by feedback to computer 100 from A/D converter 116) is greater than the set speed, system 10 will stop.

Once plunger 20 is at its rearward most position, motor 104 is actuated to move plunger 20 forward to purge all air bubbles. Pressure sensor 114 provides an indication of when one-way valve 24 is closed and pressure is beginning to build up within syringe body 18. Once the purge is completed, the total volume injected and the number of injections counter is reset.

The actuation of switch 200 also allows for full retraction and disengagement of plunger 20 from syringe body 18.

Purge switch 202 is a protected momentary push button switch. When activated, Purge switch 202 causes plunger 20 to move forward to expel air through top port 78. The forward movement of plunger 20 is limited and stopped when a predetermined pressure within syringe 18 is reached. This is sensed by pressure sensor 114. The purge operation which is initiated by Purge switch 202 will expel air within syringe 20. The user may also use Purge switch 202 to purge fluid through patient port 84 by depressing and holding Purge switch 202 continuously on.

Aspirate switch 204 is a momentary push button switch which causes computer 100 to activate pump motor 120 of peristaltic pump 44. Pump motor 120 is operated to aspirate catheter 30 at a set speed, with the aspirated fluid being collected in waste bag 52. All other motion functions are disengaged during aspiration. If the real speed of motor 120 is greater than a set speed, computer 100 will stop motor 120.

Saline switch 206 is an alternate action switch. Pump motor 120 is activated in response to Saline switch 206 being pushed on, and saline solution from bag 50 is introduced into manifold 26 and catheter 30 at a set speed. If Saline switch 206 is not pushed a second time to stop the flow of saline solution within 10 seconds, computer 100 automatically stops pump motor 120. If a time-out is reached, Saline switch 206 must be reset to its original state prior to initiating any further actions.

Enable OK switch 208 is a momentary push button switch. After the system has detected a disabling function at the end of an injection other than a limit, Enable OK switch 208 must be activated prior to activating OK switch 218 and initiating any further function.

Injection Volume Limit keys 210a and 210b are pushed to either increase or decrease the maximum injection volume that the system will inject during any one injection. Key 210a causes an increase in the maximum volume value, and key 210b causes a decrease. Once the maximum injection volume limit has been set, if the measured volume reaches the set value, computer 100 will stop motor 104 and will not restart until OK switch 218 has been depressed. If a large injection (i.e., greater than 10 ml) has been selected, OK switch 218 and Large Injection OK switch 220 must both be reset prior to initiating the large injection.

Injection Flow Rate Limit keys 212a and 212b allow the physician to select the maximum flow rate that the system can reach during any one injection. If the measured rate (which is determined by the feedback signals from tachometer 108 and potentiometer 110) reaches the set value, computer 100 will control motor 104 to limit the flow rate to the set value.

Injection Pressure Limit keys 214a and 214b allow the physician to select the maximum pressure that the system can reach during any one injection. If the measured pressure, as determined by pressure sensor 114, reaches the set value, computer 100 will control motor 104 to limit the pressure to the injection pressure limit. The injection rate will also be limited as a result.

Rise Time keys 216a and 216b allow the physician to select the rise time that the system will allow while changing flow rate during any one injection. Computer 100 controls motor 104 to limit the rise time to the set value.

In alternative embodiments. keys 210a-210b, 212a-212b, 214a-214b, and 216a-216b can be replaced by other devices for selecting numerical values. These include selector dials, numerical keypads, and touch screens.

OK switch 218 is a momentary push button switch which resets functions and hardware sensors. In response to OK switch 218 being activated. computer 100 controls display 58 to ask the operator to acknowledge that the correct function has been selected. Activation of OK switch 218 causes the status to be set to Ready.

Injection Range switch 220 is a toggle switch. Depending on whether switch 220 is in the "small" or "large" position, it selects either a high or a low injection volume range for the next injection.

Large Injection OK switch 222 is a momentary push button switch. When the large injection range has been selected by injection range switch 220, the Large Injection OK button 222 must be activated to enable OK switch 218. OK switch 218 must be activated prior to each injection. On large volume injections, the user is required to verify the volume selected by activating first Large Injection OK switch 222 and then OK switch 218.

Stop switch 224 is a momentary push button switch. When stop switch 224 is pushed, it disables all functions. Display 58 remains active.

Display panel 58 includes Set-Up display 250, Status display 252, Alerts display 254, Limits display 256, total number of injections display 260, total volume injection display 262, flow rate display 264, injection volume display 266, injection volume limit display 268, injection rate limit display 270, pressure limit display 272, rise time minimum display 274, large injection display 276, and real time clock display 278.

Set-Up display 250 contains a series of messages which are displayed as the operator goes through the set up procedure. The display of messages in set up display 250 are initiated by the actuation of set up switch 200 as described previously.

Status display 252 provides a flashing indication of one of several different operating conditions. In the embodiment shown in Fig. 4, these status conditions which can be displayed include "Ready", "Set-Up", "Injecting", "Filling", "Flushing", and "Aspirating".

Alerts display 254 and Limits display 256 notify the operator of conditions in which system 10 has encountered a critical control parameter and will disable operation, or has reached an upper or lower limit and will continue to function in a limited fashion, or has reached an upper or lower limit and will continue to operate.

Total number of injections display 260 displays the total number of injections (cumulative) given for the current patient case. The cumulative total volume injected during the current patient case is displayed by total volume display 262.

Displays 264 and 266 provide information on the current or last injection. Display 264 shows digital value of the real time flow rate to the patient during injection. Once the injection is completed, the value displayed on display 264 represents the peak flow rate reached during that injection. Display 266 shows the digital value of the volume injected during the most recent injection.

Display 268 displays the digital value of the maximum injection volume selected by operation of switches 210a and 210b. Similarly, display 270 shows the digital value of the maximum flow rate that the system will allow, as selected by switches 212a and 212b.

Display 272 shows the digital value of the maximum pressure that the system will allow to be developed in syringe 18. The pressure limit is selected by switches 214a and 214b.

Display 274 displays the minimum rise time that the system will allow while changing flow rate. The minimum rise time is selected through switches 216a and 216b.

Large injection display 276 provides a clear indication when the large injection scale has been selected by the operator.

Real-time clock display 278 shows the current time in hours, minutes, and seconds.

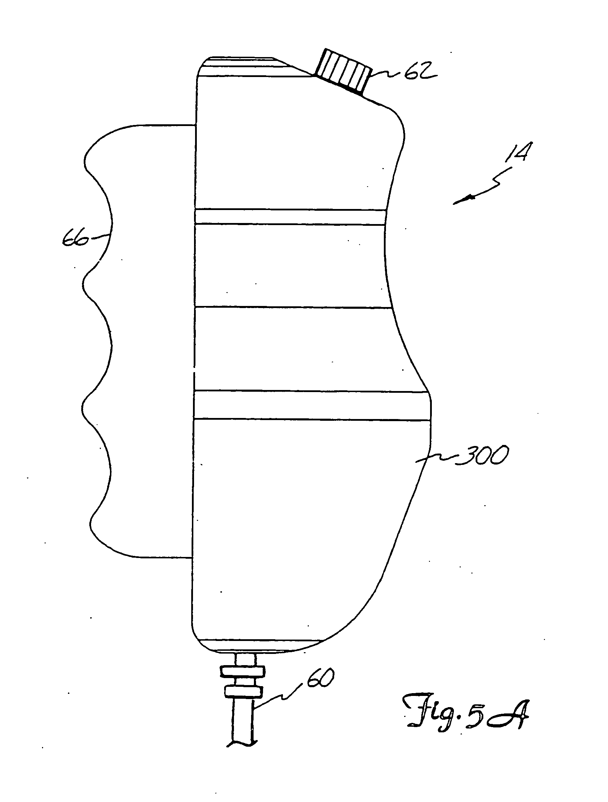

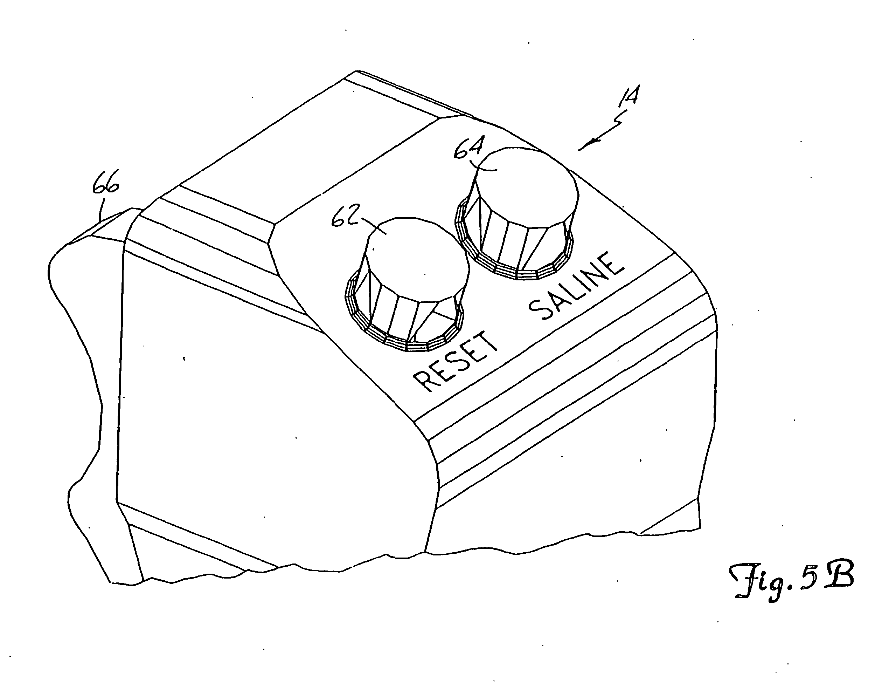

Figs. 5A and 5B show remote control 14 which includes main housing 300, which is designed to conform to the users hand. Trigger 66 is movable with respect to housing 300, and the position of trigger 66 generates a command signal which is a function of trigger position. In one embodiment, trigger 66 is linked to a potentiometer within housing 300. The command signal controls the injunction flow rate or speed. The flow rate is directly proportional to trigger position.

Reset switch 62 is a momentary push button switch whose function is identical to that of OK switch 218. Alternatively, Reset switch 62 may also be labeled "OK".

Saline switch 64 on remote control 14 is an alternate action push button switch which is pushed to turn on and pushed again to turn off. The function of Saline switch 62 is the same as that of Saline switch 206 on front panel 54.

As illustrated in another embodiment of the present invention, an alternative remote control 14' in the form of a foot pedal is used instead of the hand held remote control 14 illustrated in Fig. 1 and in Figs. 5A and 5B. Foot pedal remote control 14' includes foot operated speed pedal or trigger 66' for providing a command signal, as well as Reset or OK switch 62' and Saline switch 64'. Covers 310 and 312 protect switches 62' and 64' so that they can only be actuated by hand and not accidentally by foot. Foot pedal remote control 14' is connected to console 12 by cable 60', but could alternatively be connected by a wireless link.

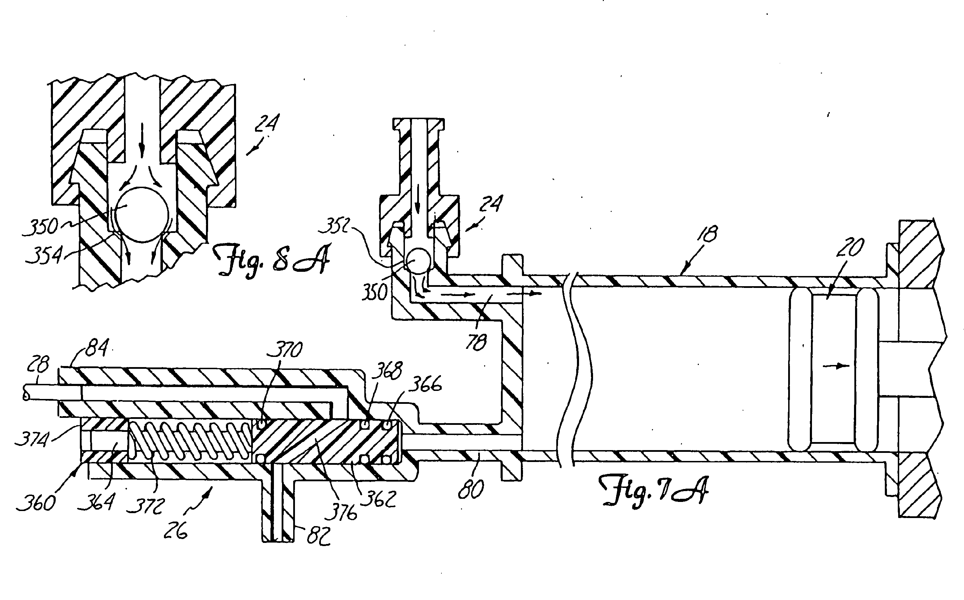

Figs. 7A-7D and Figs. 8A-8C illustrate the construction and operation of one way valve 24 and manifold 26 during Contrast Fill, Air Purge and Patient Injection operation.

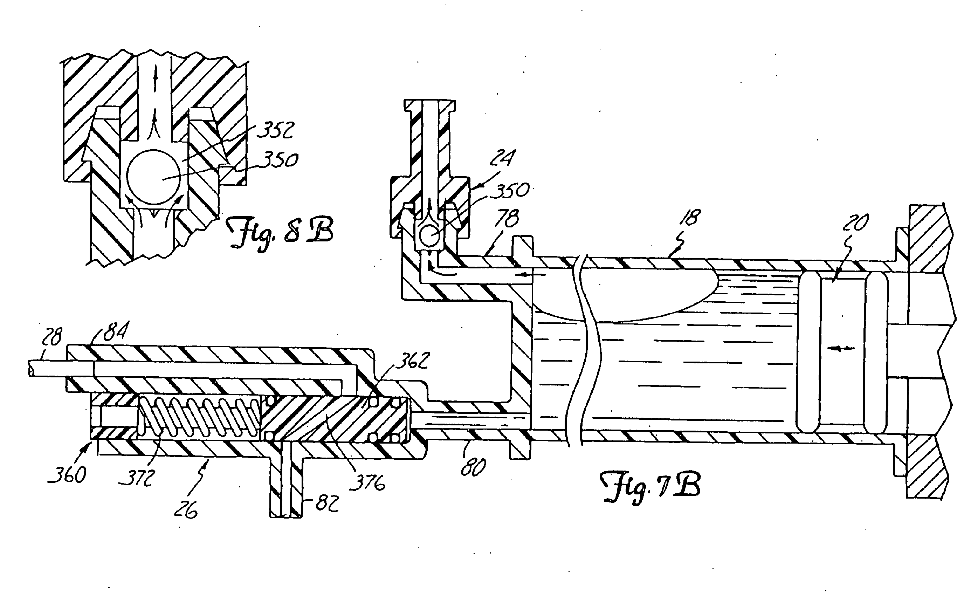

Figs. 7A and 8A illustrate one way or check valve 24, manifold 26, syringe body 18, and plunger 20 during a Contrast Fill operation. Inlet check valve of one way valve 24 includes weighted ball 350 which is positioned at its lower seated position within valve chamber 352 in Figs. 7A and 7B. Contrast material is being drawn into syringe body 18 by the rearward movement of plunger 20. The contrast material flows through passages 354 around ball 350 and into upper port 78.

Manifold 26 contains spring loaded spool valve 360, which includes spool body 362, shaft 364, O-rings 366, 368 and 370, bias spring 372, and retainer 374. As shown in Fig. 7A, during the Contrast Fill operation, bias spring 372 urges spool body 362 to its right-most position toward syringe body 18. In this position, spool body 362 blocks lower port 80 of syringe body 18 while connecting transducer saline port 82 to patient port 84 through diagonal passage 376. O-rings 366 and 368 on the one hand, and O-ring 370 on the other hand, are positioned on the opposite sides of diagonal passage 376 to provide a fluid seal.

Figs. 7B and 8B illustrate the Air Purge operation. Syringe body 18 has been filled with contrast fluid, but also contains trapped air. Plunger 20 is driven forward to force the air out of syringe body 18 through upper port 78 and through check valve 24. The force of the air may cause a slight lifting of ball 350 in check valve 20. Ball 350, however, is sufficiently heavy that the air being forced out of syringe body 18 and back toward reservoir 22 cannot lift ball 350 into its uppermost seated position where it would block the flow of air out of syringe body 18.

During the Air Purge operation, spool valve 360 is in the same position as in Fig. 7A. Diagonal passage 376 connects transducer saline port 82 with patient port 84. As a result, pressure monitoring by pressure transducer 38 can be performed during the Air Purge (as well as the Contrast Fill) operation.

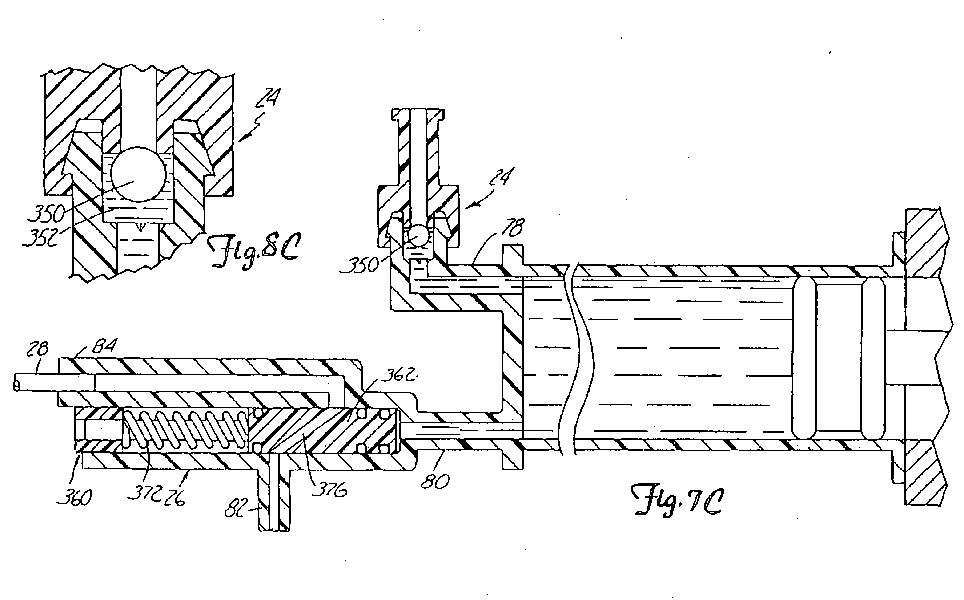

Fig. 7C and 8C illustrate the state of manifold 26 and check valve 24 at the end of the Air Purge operation and at the beginning of a Patient Inject operation.

In Fig. 7C, all air has been expelled from syringe body 18. Ball 350 floats on the radiographic contrast material, so that when all air has been removed and the radiographic contrast material begins to flow out of syringe body 18 and through upper port 78 to valve chamber 352, ball 350 is moved upwards to its upper seated position. Ball 350 blocks any continued upward flow of radiographic contrast material, as is illustrated in Figs. 7C and 8C.

In the state which is illustrated in Fig. 7C, the pressure within syringe body 18, and specifically the pressure in lower port 80 has not yet reached a level at which the bias force of spring 372 has been overcome. As a result, spool body 362 has not yet moved to the left and diagonal passage 376 continues to connect transducer saline port 82 with patient port 84.

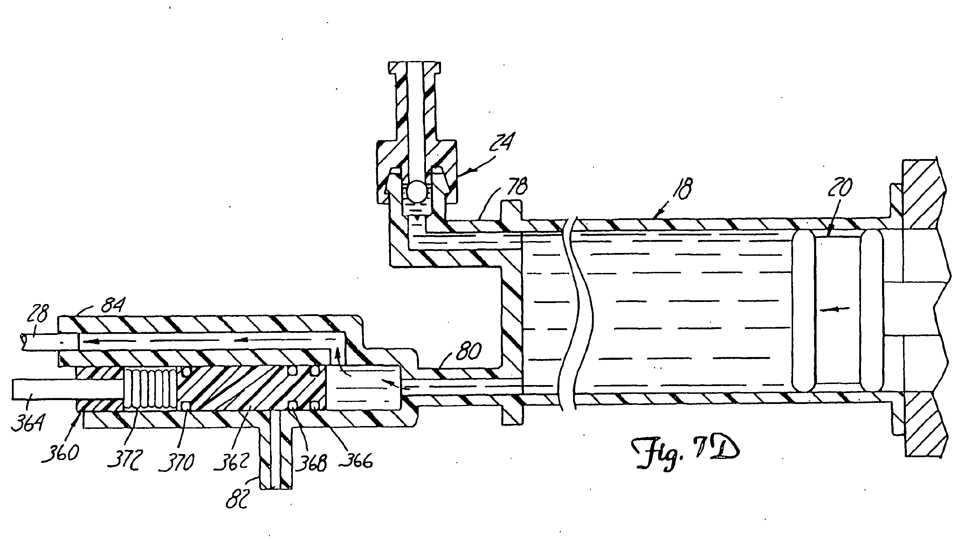

Fig. 7D illustrates the patient inject operation. Plunger 20 is moving forward, and inlet check valve 24 is closed. The pressure at lower port 80 has become sufficiently high to overcome the bias force of spring 372. Spool body 362 has been driven to the left so that lower port 80 is connected to patient port 84. At the same time spool body 362 blocks transducer/saline port 82.

By virtue of the operation of spool valve 360, the high pressure generated by movement of plunger 20 and syringe body 18 is directly connected to patient port 84, while saline port 82 and pressure transducer 38 are protected from the high pressure. The pressure to actuate may be variable and determined after manufacture by increasing or decreasing the syringe preload.

Figs. 9-11B illustrate another embodiment of the dual port syringe in the present invention. In this embodiment, conventional syringe body 400 is modified to provide dual port functionality. The modification is accomplished by adaptor insert 402 and T-connector 404.

Syringe body 400 has a cylindrical side wall 410, frustoconical end wall 412, and tubular end port 414. Adaptor insert 402, which is shown in more detail in Figs. 10 and 11 is inserted into syringe body 400 so that it mates with end wall 412 and tube 414. T-connector 404 connects to the end of tube 414, and provides upper port 420 and lower port 422.

Adaptor insert 402 has a frustoconical flange 430 and a generally cylindrical shaft 432. Flange 430 mates against the inner surface of end wall 412 of syringe body 400. Shaft 432 extends through tube 414 and through T-connector 404, so that end surface 434 of shaft 432 is generally located at the distal end of T-connector 404. Upper port groove 436 extends along the upper surface of shaft 432 and the inclined upper surface of flange 430. Upper port groove 436 stops just short of end 434.

Lower port groove 438 extends the entire length of shaft 432, along its lower surface, and then extends downward on the inclined lower surface flange 430.

When adaptor insert 402 is positioned within syringe body 400 as shown in Fig. 9, it forms a close press fit with both syringe body 400 and T-connector 404. Upper port groove 436 provides an upper port passage which extends from port 420 to the interior of syringe body 400. As shown in Fig. 9, upper port groove 436 opens into the interior of syringe body 400 at the uppermost portion of the interior.

Lower port groove 438 extends from the distal end of T-connector 404 to the lower most position in the interior of syringe body 400.

The embodiment of the present invention shown in Figures 9-11B provides an inexpensive adaptation of a conventional syringe body so that it can exhibit the advantages of dual port capability.

In conclusion, the angiographic injector system of the present invention provides interactive control of the delivery of radiographic contrast material to a catheter through a user actuated proportional control. This allows the user to adjust the flow rate of contrast material interactively as needed and as the patient's condition changes.

Although the present invention has been described with reference to preferred embodiments. workers skilled in the art will recognize that changes may be made in form and detail without departing from the spirit and scope of the invention. For example, syringe holder 16 may take other forms, such as an end loaded cylinder. Similarly, manifold 26 can take other configurations and can incorporate, for example, a part of ports 78 and 80. Subsequently a plurality of preferred aspects of the invention are highlighted, such as:

A dual port syringe comprising: a syringe body; a piston reciprocally movable in the syringe body; an upper port at a first end of the syringe body through which a medical fluid is received; and a lower port at the first end of the syringe body from which the medical fluid is delivered under pressure.

Addditionally, there may be provided a fluid reservoir for supplying fluid to the upper port; and a first valve between the fluid reservoir for permitting flow of the fluid from the fluid reservoir to the upper port.

The syringe may further comprise: a motor drive for moving the piston in a first direction away from the first end of the syringe body to cause fluid to flow from the fluid reservoir through the valve and the upper port into the syringe body, and for moving the piston in a second direction toward the first end of the syringe body to force the material out of the syringe body through the lower port.

Still further, the dual port syringe may comprise a second valve connected to the lower port for blocking flow of material out of the lower port until a predetermined pressure is reached at the lower port.

Preferably, the syringe body has a tube extending from the first end, and further comprising an insert positioned within the syringe body adjacent the first end and extending into the tube, the insert defining the upper port and the lower port.

Optionally, this injector further includes a patient port for fluid connection to a patient: and a second valve connected between the syringe and the patient port for selectively blocking and permitting flow of the medical fluid from the syringe to the patient port.

According to another option the syringe has an upper port connected to the first valve and a lower port connected to the second valve.

Preferably, the check valve has a movable element which is movable between a first position which permits flow of the medical fluid from the supply port to the syringe and a second position which prevents flow of the medical fluid from the syringe to the supply port.

The movable element may be biased toward the first position so that flow of air is permitted from the syringe to the supply port.

Optionally, the first position is below the second position and the movable element has an inherent attribute such that a force biases the movable element toward the first position. Preferably, the inherent attribute is weight and the force biasing the movable element is gravity.

Preferably, the movable element is capable of floating upward on the medical fluid from the first position to the second position when the medical fluid flows from the syringe to the check valve.

According to another embodiment there is provided an angiographic injector for injecting radiographic material into a patient, the injector comprising: an inlet port for receiving radiographic material; a pump having an upper port and a lower port; and a first valve connected between the inlet port and the upper port for permitting flow of radiographic material from the inlet port to the upper port and into the pump. and for permitting flow of air but not radiographic material from the upper port to the inlet port.

In this embodiment the first valve may be connected at the inlet port such that the inlet port is formed by the first valve.

The injector of this embodiment may further comprise a patient port for connection to a catheter; and a second valve connected between the lower port and the patient, the second valve having a first position for blocking flow of the radiographic material from the lower port to the patient port and a second position for permitting flow of the radiographic material from the lower port to the patient port.

Preferably, the second valve is connected at the patient port such that the patient port is formed by the second valve.

In said other embodiment the first valve may be a check valve. Optionally, in this case, the check valve has a movable element which is movable between a first position which permits flow of radiographic material from the inlet port to the upper port and a second position which blocks flow of radiographic material from the upper port to the inlet port.

Further, the movable element may have a weight such that flow of air from the upper port toward the supply port does not cause the movable element to move to the second position, and flow of radiographic material from the upper port toward the inlet port may cause the movable element to move to the second position and block flow of radiographic material from the check valve to the inlet port.

## Claims

1. An injector (10) for injecting medical fluid into a patient, including an inlet port (22) for receiving medical fluid, and a pump including a syringe (18, 20), the injector comprising:
a first valve (24) in fluid communication between said inlet port and said pump;
said first valve (24) having two states:
a first state which permits flow of medical fluid from said inlet port (22) into said pump (18, 20), and
a second state which permits flow of air but not medical fluid from said pump (18,20) into said inlet port (22).

2. An injector according to Claim 1, wherein said pump includes a first port (78) and a second port (80), and wherein said first valve is connected between said inlet port and said first port.

3. An injector according to Claim 2, wherein said first valve includes a check valve (24) in fluid communication between said inlet port and said first port, and wherein said check valve permitting flow of medical fluid from said inlet port to said first port and into said pump, and permitting flow of air but not medical fluid from said first port to said inlet port.

4. An injector according to claim 3, wherein:
said check valve (24) includes a movable element (350) which is movable between a first position and a second position;
said first position permitting flow of medical fluid from said inlet port to said first port; and said second position blocking flow of medical fluid from said first port to said inlet port.

5. An injector according to claim 4, wherein:
said movable element has a weight such that flow of air from said first port toward said inlet port does not cause the movable element to move to the second position, and flow of medical fluid from the first port toward the inlet port causes the movable element to move to the second position and block flow of medical fluid from the check valve to the inlet port.

6. An injector according to any one of claims 1-5, wherein said first valve is connected at said inlet port such that said inlet port is formed by said first valve.

7. An injector according to any one of claims 1-5, further including:
a patient port (84) for connection to a catheter (30); and a second valve (360) in fluid communication between said pump and said patient port;
said second valve having closed and open positions;
said closed position blocking flow of the medical fluid from said pump to said patient port;
said open position permitting flow of the medical fluid from said pump to said patient port.

8. An injector according to claim 7, wherein said second valve is connected at said patient port such that said patient port is formed by said second valve.

9. An injector according to any one of claims 2-5, further including:
a patient port for connection to a catheter; and a second valve in fluid communication between said second port and said patient port;
said second valve having first and second positions;
said first position blocking flow of the medical fluid from said second port to said patient port; and
said second position permitting flow of the medical fluid from said second port to said patient port.

10. An injector according to any one of claims 2-5 wherein:
said syringe having: a body (18), a piston (20) for reciprocal movement in said body; and
a first end (76) supporting said first port (78) through which the medical fluid is received; the first end supporting said second port (80) from which the medical fluid is delivered under pressure.

11. An injector according to claim 10, further including:
a fluid reservoir (22) for supplying medical fluid to said first port; and
a motor drive for moving said piston in a first direction away from said first end of the body to cause medical fluid to flow from the fluid reservoir through said first valve and said first port into said body; and
for moving said piston in a second direction toward said first end of the body to force the medical fluid out of said body through said second port.

12. An injector according to claim 10 wherein said body includes a tube extending from said first end, and wherein said injector further includes an insert positioned within the body adjacent to said first end and extending into said tube, the insert defining said first and second ports.

13. An injector according to any of claims 1-12, wherein said inlet port is provided at an upper port of the injector.

14. An injector according to any of claims 1-13, wherein the valve (24) is capable of withstanding static fluid pressures up to about 82.7 bar.

## Patentansprüche

1. Injektor (10) zum Injizieren medizinischer Flüssigkeit in einen Patienten,
umfassend einen Einlassanschluss (22) zum Aufnehmen medizinischer Flüssigkeit und eine Pumpe, die eine Spritze (18,20) umfasst, wobei der Injektor aufweist:
ein erstes Ventil (24) in Flüssigkeitsverbindung zwischen dem Einlassanschluss und der Pumpe;
wobei das erste Ventil (24) zwei Zustände hat:
einen ersten Zustand, der ein Fließen von medizinischer Flüssigkeit von dem Einlassanschluss (22) in die Pumpe (18,20) zulässt, und
einen zweiten Zustand, der ein Fließen von Luft, aber nicht von medizinischer Flüssigkeit von der Pumpe (18,20) in den Einlassanschluss (22) zulässt.

2. Injektor nach Anspruch 1, bei dem die Pumpe einen ersten Anschluss (78) und einen zweiten Anschluss (80) umfasst und bei dem das erste Ventil zwischen dem Einlassanschluss und dem ersten Anschluss verbunden ist.

3. Injektor nach Anspruch 2, bei dem das erste Ventil ein Rückschlagventil (24) in Flüssigkeitsverbindung zwischen dem Einlassanschluss und dem ersten Anschluss umfasst und bei dem das Rückschlagventil ein Fließen von medizinischer Flüssigkeit von dem Einlassanschluss an den ersten Anschluss und in die Pumpe zulässt und ein Fließen von Luft, aber nicht von medizinischer Flüssigkeit von dem ersten Anschluss an den Einlassanschluss zulässt.

4. Injektor nach Anspruch 3, bei dem:
das Rückschlagventil (24) ein bewegliches Element (350) umfasst, das zwischen einer ersten Position und einer zweiten Position beweglich ist;
wobei die erste Position ein Fließen von medizinischer Flüssigkeit von dem Einlassanschluss an den ersten Anschluss zulässt; und die zweite Position ein Fließen von medizinischer Flüssigkeit von dem ersten Anschluss an den Einlassanschluss blockiert.

5. Injektor nach Anspruch 4, bei dem:
das bewegliche Element ein derartiges Gewicht hat, dass ein Fließen von Luft von dem ersten Anschluss hin zu dem Einlassanschluss nicht verursacht, das bewegliche Element in die zweite Position zu bewegen, und ein Fließen von medizinischer Flüssigkeit von dem ersten Anschluss hin zu dem Einlassanschluss verursacht, das bewegliche Element in die zweite Position zu bewegen und ein Fließen von medizinischer Flüssigkeit von dem Rückschlagventil an den Einlassanschluss zu blockieren.

6. Injektor nach einem der Ansprüche 1 bis 5, bei dem das erste Ventil derart mit dem Einlassanschluss verbunden ist, dass der Einlassanschluss durch das erste Ventil gebildet wird.

7. Injektor nach einem der Ansprüche 1 bis 5, weiterhin umfassend:
einen Patientenanschluss (84) zur Verbindung mit einem Katheter (30); und ein zweites Ventil (360) in Flüssigkeitsverbindung zwischen der Pumpe und dem Patientenanschluss; wobei
das zweite Ventil geschlossene und offene Positionen hat;
die geschlossene Position ein Fließen von der medizinischen Flüssigkeit von der Pumpe an den Patientenanschluss blockiert; und
die offene Position ein Fließen von der medizinischen Flüssigkeit von der Pumpe an den Patientenanschluss zulässt.

8. Injektor nach Anspruch 7, bei dem das zweite Ventil derart mit dem Patientenanschluss verbunden ist, dass der Patientenanschluss durch das zweite Ventil gebildet wird.

9. Injektor nach einem der Ansprüche 2 bis 5, weiterhin umfassend:
einen Patientenanschluss zur Verbindung mit einem Katheter; und ein zweites Ventil in Flüssigkeitsverbindung zwischen dem zweiten Anschluss und dem Patientenanschluss;
wobei das zweite Ventil erste und zweite Positionen hat;
wobei die erste Position ein Fließen der medizinischen Flüssigkeit von dem zweiten Anschluss an den Patientenanschluss blockiert; und
wobei die zweite Position ein Fließen der medizinischen Flüssigkeit von dem zweiten Anschluss an den Patientenanschluss zulässt.

10. Injektor nach einem der Ansprüche 2 bis 5, bei dem:
die Spritze aufweist: einen Körper (18), einen Kolben (20) zur beiderseitigen Bewegung in dem Körper; und
ein erstes Ende (76), das den ersten Anschluss (78) unterstützt, durch welchen die medizinische Flüssigkeit aufgenommen wird; wobei das erste Ende den zweiten Anschluss (80) unterstützt, von welchem die medizinische Flüssigkeit unter Druck abgegeben wird.

11. Injektor nach Anspruch 10, weiterhin umfassend:
ein Flüssigkeitsreservoir (22), um dem ersten Anschluss medizinische Flüssigkeit zuzuführen; und
einen Motorantrieb, um den Kolben in eine erste Richtung, weg von dem ersten Ende des Körpers, zu bewegen, um zu verursachen, dass medizinische Flüssigkeit von dem Flüssigkeitsreservoir durch das erste Ventil und den ersten Anschluss in den Körper fließt; und
um den Kolben in einer zweiten Richtung auf das erste Ende des Körpers zu zu bewegen, um die medizinische Flüssigkeit durch den zweiten Anschluss aus dem Körper heraus zu zwingen.

12. Injektor nach Anspruch 10, bei dem der Körper einen Schlauch umfasst, der sich vom ersten Ende aus erstreckt, und wobei der Injektor weiterhin einen Einsatz umfasst, der innerhalb des Körpers an das erste Ende angrenzend positioniert ist und sich in den Schlauch hinein erstreckt, wobei der Einsatz die ersten und zweiten Anschlüsse definiert.

13. Injektor nach einem der Ansprüche 1 bis 12, bei dem der Einlassanschluss an einem oberen Anschluss des Injektors bereitgestellt ist.

14. Injektor nach einem der Ansprüche 1 bis 13, bei dem das Ventil (24) geeignet ist, statischen Flüssigkeitsdruckwerten bis etwa 82,7 bar standzuhalten.

## Revendications

1. Injecteur (10) pour injecter du fluide médical à un patient, comprenant un orifice (22) d'entrée pour recevoir du fluide médical et une pompe comportant une seringue (18, 20), l'injecteur comprenant :
une première vanne (24) en communication fluidique entre l'orifice d'entrée et la pompe ;
la première vanne (24) ayant deux états :
un premier état qui permet à du fluide médical de s'écouler de l'orifice (22) d'entrée dans la pompe (18, 20), et
un deuxième état qui permet à de l'air mais non à du fluide médical de s'écouler de la pompe (18, 20) dans l'orifice (22) d'entrée.

2. Injecteur suivant la revendication 1, dans lequel la pompe comprend un premier orifice (78) et un deuxième orifice (80) et dans lequel la première vanne est montée entre l'orifice d'entrée et le premier orifice.

3. Injecteur suivant la revendication 2, dans lequel la première vanne comprend un clapet (24) de retenue en communication fluidique entre l'orifice d'entrée et le premier orifice et dans lequel le clapet de retenue permet à du fluide médical de s'écouler de l'orifice d'entrée au premier orifice et dans la pompe et permet à de l'air mais non à du fluide médical de s'écouler du premier orifice à l'orifice d'entrée.

4. Injecteur suivant la revendication 3, dans lequel :
le clapet (24) de retenue comprend un élément (350) mobile qui est mobile entre une première position et une deuxième position ;
la première position permettant à des fluides médicaux de s'écouler de l'orifice d'entrée au premier orifice ; et à la deuxième position empêchant du fluide médical de s'écouler du premier orifice à l'orifice d'entrée.

5. Injecteur suivant la revendication 4, dans lequel :
l'élément mobile à un poids tel qu'un courant d'air allant du premier orifice en direction de l'orifice d'entrée ne provoque pas le déplacement de l'élément mobile vers la deuxième position et qu'un écoulement de fluide médical du premier orifice vers l'orifice d'entrée provoque le déplacement de l'élément mobile vers la deuxième position et empêche du fluide médical de s'écouler du clapet de retenue à l'orifice d'entrée.

6. Injecteur suivant l'une quelconque des revendications 1 à 5, dans lequel la première vanne est reliée à l'orifice d'entrée de manière à ce que l'orifice d'entrée soit formé par la première vanne.

7. Injecteur suivant l'une quelconque des revendications 1 à 5, comprenant en outre :
un orifice (84) de patient pour une connexion à un cathéter (30) ; et une deuxième vanne (360) en communication fluidique entre la pompe et l'orifice de patient ;
la deuxième vanne ayant des positions ouverte et fermée
la position fermée empêchant du fluide médical de s'écouler de la pompe à l'orifice du patient ;
la position ouverte permettant à du fluide médical de s'écouler de la pompe à l'orifice du patient.

8. Injecteur suivant la revendication 7, dans lequel la deuxième vanne est repliée à l'orifice du patient de manière à ce que l'orifice du patient soit formé par la deuxième vanne.

9. Injecteur suivant l'une quelconque des revendications 2 à 5, comprenant en outre :
un orifice de patient pour connexion à un cathéter ; et une deuxième vanne en communication fluidique entre le deuxième orifice et l'orifice du patient ;
la deuxième vanne ayant des première et deuxième positions ;
la première position empêchant du fluide médical de s'écouler du deuxième orifice à l'orifice du patient ; et
la deuxième position empêchant du fluide médical de s'écouler du deuxième orifice à l'orifice du patient ; et

10. Injecteur suivant l'une quelconque des revendications 2 à 5, dans lequel :
la seringue ayant : un corps (18), un piston (20) pour un mouvement alternatif dans le corps ; et
une première extrémité (76) supportant le premier orifice (78) par lequel le fluide médical est reçu ; la première extrémité supportant le deuxième orifice (80) duquel le fluide médical est fourni sous pression.

11. Injecteur suivant la revendication 10, comprenant en outre :
un réservoir (22) de fluide pour envoyer des fluides médicaux au premier orifice ; et
un entraînement par moteur pour déplacer le piston dans un premier sens l'éloignant de la première extrémité du corps pour faire que du fluide médical s'écoule du réservoir du fluide dans le corps en passant dans la première vanne et le premier orifice ; et
pour déplacer le piston dans un deuxième sens en direction de la première extrémité du corps pour forcer du fluide médical à sortir du corps en passant dans le deuxième orifice.

12. Injecteur suivant la revendication 10, dans lequel le corps comprend un tube s'étendant à partir de la première extrémité et dans lequel l'injecteur comprend en outre un insert placé dans le corps au voisinage de la première extrémité et s'étendant dans le tube, l'insert définissant les premier et deuxième orifices.

13. Injecteur suivent l'une quelconque des revendications 1 à 12, dans lequel l'orifice d'entrée est prévu à un orifice supérieur de l'injecteur.

14. Injecteur suivant l'une quelconque des revendications 1 à 13, dans lequel le clapet (24) est apte à résister à des pressions statiques de fluide allant jusqu'à environ 82,7 bar.
